Europäisches Patentamt

(19) European Patent Office

Office européen des brevets

(11) **EP 0 337 817 B2**

## (12) NEW EUROPEAN PATENT SPECIFICATION

(45) Date of publication and mention
of the opposition decision:
**16.12.1998 Bulletin 1998/51**

(45) Mention of the grant of the patent:
**19.01.1994 Bulletin 1994/03**

(21) Application number: **89303742.4**

(22) Date of filing: **14.04.1989**

(51) Int. Cl.[6]: **A61K 38/46**

(54) **Plasminogen activator and thrombolytic agent**

Plasminogenaktivator und thrombolytischer Wirkstoff

Activateur de plasminogène et agent thrombolytique

(84) Designated Contracting States:
**AT BE CH DE ES FR GB IT LI LU NL SE**

(30) Priority: **14.04.1988 JP 90252/88**
       **24.01.1989 JP 13044/89**

(43) Date of publication of application:
**18.10.1989 Bulletin 1989/42**

(73) Proprietor:
**KABUSHIKI KAISHA YAKULT HONSHA
Minato-ku Tokyo 105 (JP)**

(72) Inventors:
• **Sakai, Masashi
c/o Kabushiki Kaisha Yakult Honsha
Minato-ku Tokyo (JP)**
• **Matsuo, Osamu
Ohsakasayama-shi Ohsaka (JP)**
• **Watanuki, Masaaki
Kabushiki kaisha Yakult Honsha
Minato-ku Tokyo (JP)**
• **Sakurai, Toshizo**

  **Kabushiki kaisha Yakult Honsha
Minato-ku Tokyo (JP)**

(74) Representative:
**Sheard, Andrew Gregory et al
Kilburn & Strode
20 Red Lion Street
London WC1R 4PJ (GB)**

(56) References cited:
**EP-A- 0 077 664**

• **Chemical Abstracts, vol. 86, no. 11, March 14, 1977, p. 202, abstract no. 67386z, Columbus, Ohio, US; R. Eriksson: "The mechanism of activation of plasminogen by staphylokinase"; & ZENTRALBL., PARASITENKD., INFEKTIONSKR. HYG., ABT. 1, SUPPL. 1976, 5(Staphylococci Staphylococcal Dis., Proc. Int. Symp., 3rd 1975), 523-528**
• **Biological Abstracts, vol. 81, 1986, p. AB748, abstract no. 65436, Philadelphia, PA, US; K. Kanae: "Fibrinolysis by staphylokinase in vivo"; & TOKYO JIKEIKAI MED., vol. 100, no. 5 (1985), pages 925-934**
• **Chemical Abstracts, vol. 84, no. 13, March 1976, p. 317, abstract no. 87381f, Columbus, Ohio, US; B. Kowalska-Loth et al.: "The activation by staphylokinase of human plaminogen"; & ACTA BIOCHEM. POL. 1975 22(4), 327-339**
• **J. Biol. Chem., vol. 268, pp. 9811-9816, 1993**
• **Blood, vol. 84, pp. 680-686, 1994**
• **J. Biol. Chem., vol. 266, pp. 11826-11832, 1993**
• **Fibrinolysis, vol. 6, pp. 214-225, 1992**
• **Eur. J. Biochem., vol. 211, pp. 91-97, 1993**
• **Thromb. Haemostas., vol. 70, pp. 326-331, 491-494, 1993**
• **Fibrinolysis, vol. 7, pp. 242-..., 1993**
• **Circulation, vol. 87, pp. 1850-1853, 1993**

Remarks:
The file contains technical information submitted after the application was filed and not included in this specification

EP 0 337 817 B2

Printed by Xerox (UK) Business Services
2.16.6/3.4

## Description

This invention relates to a plasminogen activator and a thrombolytic agent.

Conventionally, plasminogen activators such as urokinase, which is primarily synthesized in the kidney and excreted in urine (hereinafter referred to as UK), and streptokinase, which is a metabolite of ß-haemolytic streptococci (hereinafter referred to as SK), have been clinically used.

SK is less expensive than UK and is commonly used in Europe and USA; because UK and SK activate plasminogen in circulating blood (in the liquid phase) to degrade fibrinogen as well degrading fibrin in thrombi (solid phase) as their primary objective, side effects such as apoplexy cause problems. Therefore, these substances are generally administered through a catheter directly into the site where the thrombus is formed and, for that reason, considerable clinical facilities have been required.

The antigenicity of SK also causes problems because SK is a protein from a bacterium.

Therefore, the development of new thrombolytic agents which allow the degradation of fibrin selectively has been awaited; these agents may differ from conventional thrombolytic agents in their ability to degrade fibrinogen in blood.

Recently, tissue plasminogen activator (hereinafter referred to as tPA) and prourokinase (hereinafter referred to as Pro-UK) have been discovered in response to the aforementioned need and they have caught attention as thrombolytic agents possessing fibrin specificity.

On the other hand, experiments on the administration of the complex between SK and plasminogen have also been attracting attention in the clinical field (US-A-4178368, US-A-4082612). The complex works firstly to prevent inhibitory effects of high-level SK and, secondly, to mask the antigen site of SK in order to decrease its antigenicity by binding SK to plasminogen. Another experiment to acylate the active centre of SK-plasminogen complex (EP-A-0028489) was carried out so as to enhance the fibrin specificity of the complex by deacylation at the site of the thrombus; the experiment was also effective to prevent autodigestion of the complex.

Moreover, recent recombinant DNA techniques have enabled the production of staphylokinase (hereinafter referred to as SAK) using Escherichia coli on a large scale (EP-A-0077664, US-A-4532211, Sato, T. Eur. J. Biochem, 149; 557-63 (1985)).

SAK, like SK, may be supposed to bind to plasminogen to form a complex, which functions as a plasminogen activator. However, the synthesis and isolation of the complex and its activity have not yet been reported. In addition, SAK, a thrombolytic agent, is different from SK in its amino acid sequence and antigenicity, and has a molecular weight of one third or less that of SK (Nucleic Acid Research, 11, pp. 7679-93 (1983)).

But SAK, like PA and Pro-UK described above, causes problems in that it has a short half-life in living organisms and is rapidly inhibited by plasminogen activator inhibitor (hereinafter referred to as PAI) (Collen, D. et al. Thrombos. Haemostas, 52; 24-26, '84), and, therefore, it cannot produce the fruitful results that were initially expected (Sherry, S. New England J. Med. 313; 1014-17, '85).

The present inventors have elucidated the difference in the mechanism of action between SAK and SK as a result of extensive efforts and have confirmed that SAK is a superior thrombolytic agent with fewer side effects, such as apoplexy.

The disadvantages of SAK, in that SAK requires about 30 minutes of lag time to express its fibrinolytic activity and that high-level SAK has inhibitory effects on fibrinolysis, became evident at that time.

Routine thrombolytic treatments should be able to be carried out in an emergency and optimum doses of thrombolytic agents should be also established. These needs suggest that the lag time in SAK exhibiting its effects and the difficulty of determining the reason for seeing no clinical effects (whether it may be caused either by an excess, or by a shortage, of the dosage because of inhibitory effects of the high-level SAK), would be disadvantages of SAK.

The present invention provides a plasminogen activator and a thrombolytic agent, containing SAK as the principal ingredient, which have fewer side effects (such as apoplexy), which have improved stability and which are not liable to be inhibited by PAI, thus resulting in prolonged activity in living organisms.

In order to achieve the aforementioned objective, a first aspect of the invention provides a thrombolytic agent containing SAK as an active ingredient.

A second aspect of the invention provides a thrombolytic agent, namely an SAK-plasminogen (or plasmin) complex, which functions as a plasminogen activator.

A third aspect of the invention provides a thrombolytic agent containing an SAK-plasminogen complex as an active ingredient.

In accordance with the present invention, SAK forms a complex with plasminogen and the complex functions as a plasminogen activator (Kowalska-Loth, B. & Zakrzewski, K. Acta Biochim. Pol., 22; 327-39 (1975)).

The mechanism of action of SAK is similar to that of SK in these points but the studies of the inventors demonstrated several different points in the feature between SAK and SK. That is,

a) The plasminogen activator potential of SAK is enhanced in the presence of fibrin. Thus, SAK has a high specificity to fibrin. On the other hand, the enhancement of the plasminogen activator potential of SK in the presence of fibrin is weak.

b) In circulating blood, the reaction of SAK-plasminogen complex is inhibited by $\alpha_2$-plasmin inhibitor ($\alpha_2$-antiplasmin, hereinafter referred to as $\alpha_2$-PI), while on fibrin the reaction is not likely to be inhibited by $\alpha_2$-plasmin inhibitor; therefore, it is expected that the reaction on fibrin moves selectively toward fibrinolysis (thrombolysis). By contrast, the reaction of SK-plasminogen complex is not inhibited by $\alpha_2$-PI. This results in the degradation of fibrinogen in circulating blood to cause such side effects as apoplexy.

c) SK is rapidly degraded by plasmin, while SAK is not so readily degraded by plasmin; this results in the good stability of SAK in plasma.

d) Since the molecular weight of SAK is approximately 15,000 (Nucleic Acid Research, 11, pp. 7679-93 (1983)), about one third that of SK, the permeability of SAK into thrombi appears to be good.

The present invention demonstrates that the mechanism of action of SAK should be represented as in Fig. 1, based on general considerations of its feature as described above.

Figure 1 is an explanatory drawing illustrating the difference in the mechanisms of action between SAK and SK, which was analyzed by the inventors. In the figure, SAK represents SAK; SK, SK; $\alpha_2$-PI, $\alpha_2$-plasmin inhibitor; Fn, fibrin; Plg, plasminogen and Plm, plasmin.

The SAK-plasminogen complex formed in bloodstream is rapidly inhibited by $\alpha_2$-PI to cause inactivation of the plasminogen (or plasmin) and SAK is not inhibited and the plasminogen therein is activated into plasmin, resulting in fibrinolysis. Conversely, in the case of SK, the SK-plasminogen (or plasmin) complex formed in the bloodstream is not inhibited by $\alpha_2$-PI to cause a great amount of plasmin in the bloodstream, resulting in the degradation of fibrinogen (not shown in figure). This is the reason why SK is accompanied with a high incidence of apoplexy.

The lag time needed for the expression of the SAK activity, which is regarded as a disadvantage of SAK, may be due to the time required for the expression of protease activity resulting from the change in the plasminogen conformation after SAK binds to the plasminogen (Acta Biochimica Polonica 212, pp. 329-31 (1975)).

The inhibitory action of SAK at a high concentration appears to be caused by the binding of most of the plasminogen (or plasmin) in the bloodstream to SAK, resulting in the depletion of unbounded plasmin with the activity of fibrinolysis.

These problems may be solved by premixing and incubating SAK and plasminogen for a given time. That is, the SAK-plasminogen (or plasmin) complex with a molar ratio of 1:1 is produced by this procedure and, since the complex has protease activity, it is useful as a plasminogen activator as it is. In addition, since all the SAK is present as a complex with plasminogen (or plasmin), it does not bind plasminogen (or plasmin) any more and, therefore, does not inhibit fibrin degradation even at high SAK concentrations. SK-plasminogen complex is likely to autodegrade, while the SAK-plasminogen (or plasmin) complex does not readily autodegrade and so it is stable as it is for a long period of time.

The SAK-plasminogen (or plasmin) complex has a plasminogen activating activity. That is, the complex degrades plasminogen restrictively to change it into plasmin. Plasmin degrades fibrin in thrombi, leading to fibrinolysis. Thus, the SAK-plasminogen (or plasmin) complex, like UK and SK, is effective in treating various thrombotic diseases including thrombosis itself.

This invention is exemplified in detail in the following examples and demonstrates that SAK is useful as a thrombolytic agent having fewer side effects such as apoplexy, because of its mechanism of action: SAK has a high specificity to fibrin as compared to SK and the SAK-plasminogen (or plasmin) complex formed in the bloodstream is rapidly inhibited by $\alpha_2$-PI so that plasminogen is not activated, whereas the complex between plasminogen (or plasmin) bound to fibrin and SAK is not inhibited, so that plasminogen is activated into plasmin to cause thrombolysis.

Additionally, the stability of SAK in blood is better than that of other plasminogen activators such as SK, and the mechanism of action of SAK differs from that of tPA or pro-UK, as is shown in Figure 1; so SAK is not readily inhibited at thrombus sites by PAI, an inhibitor to tPA and pro-UK, and retains its activity in living organisms for a long period of time.

Thus, SAK enables the fibrinolytic activity in blood to be increased so that it appears to be effective in chronic thrombotic disease treatment.

Furthermore, SAK has such a smaller molecular weight than other conventional thrombolytic agents that it permeates into thrombi better.

On the other hand, the SAK-plasminogen (or plasmin) activator is an activated plasminogen activator itself and, therefore, it acts more rapidly than SAK alone. Also, since all the SAK is present as a complex with plasminogen (or plasmin), even it use at high concentrations does not inhibit fibrinolysis. Additionally, the complex between SAK and plasminogen (or plasmin) is more stable than the corresponding SK-plasminogen complex so that it autodegrades less. Because most of the antigenic sites of SAK are masked, its antigenecity may be expected to be less than SK.

The invention is illustrated by the following examples.

Example 1. <u>Antigenicity test of SAK and SK</u>

Rabbits were immunized to prepare polyclonal antibodies to SAK; cross-reactivity between the antibodies and SK was tested by the Ouchterlony method.

The result was that no precipitation-line between anti-SAK antibody and SK formed, showing that the antigenecities of the two proteins were different.

Example 2. <u>Analysis of the reaction mechanism of SAK</u>

The SAK-plasminogen (or plasmin) complex formed in blood is rapidly inhibited by $\alpha_2$-PI so that the plasminogen is not activated, whereas the complex between SAK and plasminogen (or plasmin) bound to fibrin is not inhibited so that the plasminogen is activated into plasmin to cause thrombolysis. Conversely, in the case of SK, the SK-plasminogen (or plasmin) complex formed in bloodstream is not inhibited by $\alpha_2$-PI, so that a large amount of plasmin is formed in bloodstream thereby causing the degradation of fibrinogen. This is assumed to be a cause of the high incidence of apoplexy associated with treatment by SK. The analysed SAK mechanism of action is sequentially explained in the following sections.

2-1. <u>Studies on fibrin specificity in a closed-circuit model</u>

The closed-circuit system, the closest model to living organisms for examining fibrin specificity of thrombolytic agents (or plasminogen activator) was developed by Matsuo <u>et al</u>. (Matsuo, O. <u>et al</u>.: <u>Thrombos. Res</u>., 24; 347-58, (1981)).

The fibrin specificity of SAK was examined in the closed-circuit model (a model close to living organisms).

<u>Methods</u>

(1) $^{125}$I-labelling of fibrinogen.

Human fibrinogen (Plasminogen Free, manufactured by Sigma Co. Ltd.) was radio-labelled with $^{125}$I by the chloramine T method and the labelled fibrinogen was purified by gel filtration. 1.5 ml of $^{125}$I-labelled fibrinogen at about $3.5 \times 10^{-6}$ cpm/ml was obtained.

(2) Preparation of $^{125}$I-labelled thrombus.

$^{125}$I-fibrinogen (20 $\mu$l) was added to 1 ml of human plasma and mixed with 100 $\mu$l of 25 mM calcium chloride and then 50 $\mu$l of the solution of thrombin (100 U/ml; Sigma Co. Ltd.), an enzyme converting fibrinogen into fibrin, was added to form an $^{125}$I-labelled thrombus.

(3) Thrombolytic experiments in a closed-circuit model.

Human plasma 1 (total volume of 21 ml) was circulated at a flow rate of 1.95 ml/min within a lower chamber 3, a peristaltic pump 4 and an upper chamber 5 into which a labelled thrombus 6 was put; all the chambers were connected by silicon tubing. 1 ml of a sample under test 7 (SAK or SK) was dropped into the lower chamber 3 and the remaining 5 ml portion was continuously added using the peristaltic pump 8 (flow rate 0.04 ml/min). The reaction system was kept at 37°C and every hour circulating plasma was removed from the lower chamber 3 for assays of radioactivity and plasma components.

(4) Measurement of fibrinolytic ratio.

The radioactivity of the plasma was measured by a gamma counter and the measured values were used to calculate the radioactivity of the whole circulating plasma; the ratio of the radioactivity compared to that of the labelled thrombus was represented as the fibrinolytic ratio as a percentage.

(5) Quantitative determination of plasma fibrinogen.

To a plasma sample of 50 $\mu$l was added 200 $\mu$l of barbital buffer (pH 7.75) containing 50 mM calcium chloride. An aliquot (50 $\mu$l) of the resulting solution was taken into a 96-well microtiter plate. 50 $\mu$l of thrombin solution (100 U/ml; Sigma Co. Ltd.) was added to the sample solution and allowed to react at 37°C for 10 min, absorbance at 405 nm was measured. Fibrinogen concentrations (%) were proportionally calculated by expressing the absorbance of the solution to which water was added instead of thrombin as 0% and the absorbance of the solution in which normal plasma was coagulated as 100%.

The fibrinolytic specificity herein as represented by the following formula:

$$(\text{Fibrinolytic specificity}) = \frac{(\text{Fibrinolytic ratio (\%)})}{(\text{Fibrinogen degradation ratio (\%)})}$$

(6) Quantitative determination of plasma plasminogen.

A sample (36 $\mu$l) diluted 40 times with 50 mM Tris-HCl buffer (pH 7.4) was taken in the 96-well microtitre plate and 14 $\mu$l of urokinse (5000 IU/ml) was added to each well and incubated at 37°C for 15 min to form plasmin. Then, 10 $\mu$l of the synthetic chromogen CBS 33.08 (7.5 mM; Stago, Co. Ltd.) was added, and the mixture was incubated at 37°C for 15 min; this was followed by the addition of 2% citrate solution (200 $\mu$l) to terminate the reaction. Absorbance at 405 nm was measured.

The concentrations of plasmin were proportionally determined based on the measured absorbance of normal plasma (100%) and 4-fold diluted plasma (25%).

(7) Quantitative determination of plasma $\alpha_2$-plasmin inhibitor ($\alpha_2$-PI).

A sample (28 $\mu$l) diluted 40 times with 50 mM Tris-HCl buffer (pH 7.4) containing 120 mM monomethylamine hydrochloride was taken in the 96-well microtitre plate and 22 $\mu$l of plasmin (16.7 $\mu$l/ml) was added to each well and incubated at 37°C for 15 min to form a complex with plasma $\alpha_2$-PI. Then, 10 $\mu$l of the synthetic chromogen CBS 33.08 (7.5 mM; Stago Co. Ltd.) was added thereto, incubated at 37°C for 15 min and this was followed by the addition of 2% citrate solution (200 $\mu$l) to terminate the reaction. The absorbance at 405 nm was measured.

The concentrations of $\alpha_2$-PI (%) were proportionally determined based on the measured absorbance of normal plasma (100%) and 4-fold diluted plasma (25%) in a similar manner to the case of plasminogen.

Results and Discussion.

SAK barely degrades fibrin at 0.625 $\mu$g/ml, while at 1.25 $\mu$g/ml or more it degraded fibrin dose-dependently; at 10 $\mu$g/ml, almost the maximal activity for degrading fibrin was attained. On the other hand, SK degraded fibrin at 1.25 $\mu$g/ml or more, and at 10 $\mu$g/ml, the maximal activity for degrading fibrin was reached. The two enzymes degraded fibrin dose-dependently, but the specific activity of SAK was higher than that of SK. For example, 5 $\mu$g/ml of SAK was equal to 20 $\mu$g/ml of SK in specific activity.

In addition, an apparent difference was observed in the reaction curves of the two enzymes. The time course with SK is linear, while at an early phase with the reaction of SAK, fibrinolysis was barely observed, but the reaction proceeded rapidly once degradation was initiated.

The metabolism of fibrinogen in circulating blood will now be described. SK degrades fibrinogen significantly, while the degradation of fibrinogen by SAK is slight and, in particular at the concentration of 2.5 $\mu$g/ml, it was observed that 100% of fibrin degraded after 6 hours of reaction, in spite of 85% of fibrinogen remaining; this indicates that SAK has a high specificity to fibrin.

It is apparent that SAK has a higher potential for fibrin degradation and a lesser potential for fibrinogen degradation than SK.

In the same experiments, other fibrinolytic agents such as t-PA and pro-UK did not show a fibrinolytic specificity exceeding 3 and these results indicate that SAK is a substance having superior fibrin specificity.

Thus, SAK is indicated to have a high fibrinolytic specificity in the closed-circuit model (a model close to living organisms) as it lysed a plasma clot (artificial thrombus) at a lower concentration than SK and degraded less fibrinogen. Furthermore, the model also indicates that SAK has a lesser potential for reducing plasminogen and $\alpha_2$-PI, and that the plasminogen activating reaction by SAK is mild in plasma.

2-2. Evaluation of fibrin specificity caused by the addition of thrombin in human plasma

Fibrinogen was converted into fibrin by the addition of thrombin to human plasma and, thereby, the mechanism of plasminogen activation was analysed.

Methods.

Tris-HCl buffer (0.1 M, 150 µl; pH 7.4) was taken in a 96-well microtitre plate and 20 µl of human plasma (Ci-Trol, manufactured by Midori Juji Co. Ltd.) and 10 µl of the synthetic chromogen S-2251 (5 mg/ml; manufactured by kabi-Vitrum Co. Ltd.) were added thereto, followed by the addition of 10 µl of the human-thrombin solution or water as a control. SAK or SK (10 µl) at a given concentration was added to initiate the reaction. The reaction was carried out at 37°C and changes in absorbance at 405 nm were measured.

Results and Discussion.

The plasminogen activating reaction of SAK was greatly enhanced by the addition of thrombin. On the other hand, the reaction by SK was also enhanced by thrombin but the degree of the enhancement by SK was weak; for example, in comparison between the activity of SAK of 40 µg/ml and that of SK of 10 µg/ml, the two activities were almost equal in the presence of thrombin, while in its absence, the activity of SK was approximately 10 fold that of SAK.

Thus, the SAK activity was indicated to be greatly enhanced by thrombin (i.e. in the presence of fibrin) and to have a high fibrin specificity.

2-3. Enhancement of the SAK reaction by fibrin in the presence of $\alpha_2$-PI

The actions of SAK and SK were measured on the presence of $\alpha_2$-PI and fibrinogen and the effects of thrombin on the fibrin formation were examined.

Methods.

SAK or SK solution (10 µl) was taken in a microtube and 10 µl of the human-thrombin solution (20 units/ml; Sigma Co. Ltd.) was added thereto, followed by addition of the mixed solution consisting of 50 mM Tris-HCl buffer (140 µl, pH 7.4, 0.01% Tween 80™ added), human fibrinogen (10 µl, 3.0 mg/ml; Plasminogen Free, manufactured by Sigma Co. Ltd.), human plasminogen solution (10 µl, 0.15 mg/ml; glutamyl-type, manufactured by Kabi-Vitrum Co. Ltd.), human $\alpha_2$-PI solution (10 µl, 0.06 mg/ml; manufactured by Protogen Co. Ltd.) and the synthetic chromogen S-2251 (10 µl, 10 mM; manufactured by Kabi-Vitrum Co. Ltd.) to initiate the reaction. After the incubation at 37°C for 30 min, the reaction was terminated by the addition of 8% citrate solution (50 µl). An aliquot (200 µl) of the resulting solution was taken in each well of 96-well titre plate and the change in the absorbance at 405 nm was measured by a photometer for titre plate.

In the case of gelation of the reaction solution by the addition of thrombin, the resulting gel was dissolved by the addition of citrate solution and shaking.

Results and Discussion.

The activities of SAK and SK were not affected by the addition of thrombin in the absence of $\alpha_2$-PI and, therefore, it was indicated that the two did not have fibrin specificity under these reaction conditions.

However, SK was observed to have almost equal activities in the presence of $\alpha_2$-PI, although they were rather weak compared with those in the absence of $\alpha_2$-PI; by the addition of thrombin, the activities restored about 2-3 fold following the addition of thrombin.

Conversely, SAK did not exhibit the activity even at as high a concentration as 5.0 µg/ml without thrombin, and inhibition by $\alpha_2$-PI was observed.

A more important change is that the activity of SAK was much more greatly enhanced by the addition of thrombin than that of SK; for example, SAK at 5.0 µg/ml showed no absorbance change without thrombin while it showed an absorbance change as high as 0.93 with thrombin added. This indicates that the inhibition of the SAK reaction by $\alpha_2$-PI is restored by the fibrin formation.

Thus, the finding that SAK does not have a fibrin specificity in the absence of $\alpha_2$-PI and exhibits fibrin specificity only in the presence of $\alpha_2$-PI indicates that $\alpha_2$-PI is strongly involved in the expression of fibrin specificity for SAK.

2-4. Effects of SAK and SK on the plasmin inhibitory reaction by $\alpha_2$-PI

In order to examine more explicitly the involvement of $\alpha_2$-PI in the expression of fibrin specificity of SAK as is shown in section 2.3, the effects of SAK and SK on the plasmin inhibitory reaction by $\alpha_2$-PI were examined.

Methods.

10 µl of each of solutions of SAK or SK (0-50 µg/ml each was added to 50 µl of a human-plasmin solution (0.05 C.U./ml; manufactured by Sigma Co. Ltd.) in a 96-well assay plate. After the mixture allowed to stand at room temperature for 5 min, 10 µl of $\alpha_2$-PI (0-5 I.U./ml; manufactured by Protogen Co. Ltd.) was added thereto, and followed by the addition of the mixture solution (130 µl) of 50 mM Tris-HCl buffer (120 µl, pH 7.4) and the synthetic chromogen S-2251 (10 µl, 10 mM; manufactured by Kabi-Vitrum Co. Ltd.) to initiate the reaction. The reaction was done at 37°C for 30 min, and the changes in the absorbance at 405 nm were measured by a photometer for assay plates.

The inhibition rate was calculated according to the following formula; herein, the plasmin activity without $\alpha_2$-PI is represented as A and that with $\alpha_2$-PI is represented as B.

$$\text{The inhibition rate (\%)} = (A-B)/A \times 100.$$

Results and Discussion.

80% of the inhibition rate was observed at the time of the addition of $\alpha$2-PI (5 I.U./ml) and the inhibition rate decreased accompanied by the restoration of plasmin activity when the concentration of SAK and SK increased. In this case, intense restoration of activity was observed for SK and, furthermore, no inhibition was observed following the addition of SK at a concentration of 50 µg/ml; on the other hand, SAK at the same concentration showed an inhibition rate of 60%, which indicated that SAK weakly restores inhibitory activity to plasmin.

There have been some reports about the inhibition of the plasmin inhibitory reaction of SK by $\alpha_2$-PI (S.A. Cederholm-Williams et al, Eur. J. Biochem. 100, PP. 125-132, '79) but no reports have been published about the relation between SAK and $\alpha_2$-PI.

Although the restoration mechanism of the plasmin activity by SK is not certain, there may be a possibility that SK binds to the L(B) chain, resulting in steric inhibition of the binding of plasmin to the active centre of $\alpha_2$-PI. SAK with a molecular weight of about one third that of SK may cause little steric effects although it does bind to the L(B) chain similarly.

$\alpha_2$-PI is a proteinaceous inhibition factor with plasmin specificity and it rapidly inhibits plasmin formed in the bloodstream; however, plasmin bound to fibrin is saturated at the lysine binding site (hereinafter referred to as the LBS) thereof and, therefore, it cannot bind to $\alpha_2$-PI through the LBS, resulting in no inhibition (B. Wiman et al. Biochem. Biophys. Acta., 579, 142-154, '79, etc.).

This suggests that $\alpha_2$-PI does not inhibit plasmin formed on the thrombus but rather the plasmin formed in bloodstream, resulting in the selective degradation of fibrin.

Accordingly, it has been elucidated that SAK has the fibrin specificity corresponding to the regulatory function of $\alpha_2$-PI.

So, the SAK-plasminogen (plasmin) complex formed in the bloodstream is rapidly inhibited by $\alpha_2$-PI, and plasminogen is not activated, but because the complex between the plasminogen (or plasmin) bound to fibrin and SAK is not activated, the activation of plasminogen into plasmin causes thrombolysis. Conversely, it is assumed that, in the case of SK, the SK-plasminogen (or plasmin) complex formed in bloodstream is not inhibited by $\alpha_2$-PI, resulting in the formation of a large amount of plasmin to degrade even fibrinogen (not shown in the figures).

Thus, the mechanism of action indicates that SAK reaction is inhibited by $\alpha_2$-PI, while the reaction proceeded selectively on fibrin because $\alpha_2$-PI did not readily affect the reaction thereon and that SAK has acquired a higher specificity to fibrin than SK.

In other words, it is confirmed that the SAK reaction is inhibited by $\alpha_2$-PI in free-form plasminogen but it proceeds selectively if the plasminogen is bound to fibrin because the LBS in the plasminogen is saturated and $\alpha_2$-PI cannot react (Wiman, B. et al.: Biochem. Biophys. Acta, 579; 142-54. '79).

As has been described above, SAK has a higher specificity to fibrin and the fibrinogen degradation is inhibited in circulating blood and apparently it can be used as a thrombolytic agent with fewer side effects (such as apoplexy); these have been a problem with conventional thrombolytic agents.

Example 3. Stability of SAK in plasma

SAK or SK was added to human plasma and incubated at 37°C and an aliquot of the resulting solution was subjected to the SDS-polyacrylamide gel electrophoresis in order to examine the fibrinolytic activity by fibrin autography.

As a result, SAK did not lose its activity even after incubation for 20 hours, but SK completely lost its activity. Similar results were obtained by the use of human-plasminogen instead of plasma, showing the stability of SAK.

Example 4. The SAK-plasminogen (or plasmin) complex.

The reaction of the preformed SAK-plasminogen (or plasmin) complex was examined in order to improve the lag time required for the expression of the activity.

4-1. The formation of SAK-plasminogen (plasmin) complex.

To 2 ml of human plasminogen solution (Seikagaku Kogyo Co. Ltd. 0.6 mg/ml) dissolved in 0.1 M sodium phosphate buffer (pH 7.4) was added 86 $\mu$l of the SAK solution dissolved in the same buffer (2.4 mg/ml) in a molar ratio of 1:1 to incubate at room temperature. By this method the SAK-plasminogen (plasmin) complex was formed possessing an active centre, which was subsequently applied to gel filtration on a SUPEROSE 12 H/R 10/30 column (FPLC system). The SAK-plasminogen complex was eluted at a high molecular region thereby to separate and purify it from the SAK which is unbound to plasminogen.

4-2. Time course of fibrinolysis by SAK-plasminogen (or plasmin) complex.

The fibrinolytic activity of the complex obtained by the aforementioned method was measured by the standard fibrin plate method. That is, 80 mg of human-fibrinogen (Kabi Co. Ltd., grade L) was dissolved in 20 ml of physiological saline (adjusted to pH 7.4 with barbital-acetate buffer) and centrifuged to remove the undissolved fraction. The resulting solution (8 ml) was taken in a dish of a diameter of 8 cm and 0.3 ml of human-thrombin (3.3 IU/ml) was added thereto to coagulate fibrinogen. The sample was added dropwise with a micropipette on the fibrin plate prepared in this manner, incubated at 37°C and observed for fibrinolysis.

Lytic spots were observed as late as 60 min after the initiation of the reaction with SAK alone (A); in the case of the SAK-plasminogen complex (B), lytic activity was observed 15 min later and the activity was clearly observed 30 min later; these results indicate the rapid action of SAK.

On the other hand, lytic spots were observed 30 min later in the case of the SK alone and in the case of the SK-plasminogen complex, and the two showed almost equal activities.

Thus, it was demonstrated that, by complexing with plasminogen, the lag time of SAK can be induced and the rapidity of action of SAK can equal or better that of SK.

Example 5. Changes in the fibrinolysis by the concentrations of SAK-plasminogen (plasmin) complex.

Fibrinolytic reactions were compared between concentrates of SAK alone, SK alone, the plasminogen-SAK complex and the plasminogen-SK complex.

Lytic spots are observed in a double-ring with SAK and SK (A, C) at high concentrations of 5 $\mu$M; this indicates that the lytic reaction was inhibited in the centre of the spot that is, at the regions of the high concentration). On the other hand, there was no sign of any inhibition of lytic reaction in the case of the plasminogen complexes shown as B and D, and even the centres of all the spots of the two were lysed.

In addition, the activity of SAK alone (A) at a low concentration of 0.04 $\mu$M showed a weak lytic activity, while that of the plasminogen complex (B) at an equal concentration showed an activity comparable to that at higher concentrations; in particular, in the case of SAK, the complex thereof with plasminogen showed efficacy in a wide range of concentrations.

The activity of SK alone (C) at low concentration showed an apparent lytic spot and the complex thereof with plasminogen did not lead to the enlargement of the effective range of concentrations.

6. Toxicity test

A toxicological test was done; SAK at a dose of 1 mg/kg was intravenously administered to 10 male ICR mice weighing 28-35 g each. No mice died as a result.

As has been described above, SAK has superior stability in blood compared to plasminogen activators such as SK and it is barely inhibited by PAI, by analogy of the mechanism of action that its complex with plasminogen or plasmin might activate plasminogen resulting in prolonged retention of activity in living organisms. Accordingly, the fibrinolytic activity of SAK can constantly increase and, therefore, it is expected to be effective for chronic thrombotic diseases.

In addition, as is shown in Example 4, SAK preincubated with plasminogen makes the lag time shorter and leads to rapidity of action. SAK mixed with plasminogen in an equimolar ratio of 1:1 does not show such inhibitory action at high concentration; the complex with SAK increases the activity at lower concentrations and is shown to be effective in a wider range of concentrations than SAK alone. Thus, it was demonstrated that as a fibrinolytic agent SAK turns out to be a rapidly and topically effective drug with a wide dosage range if used in the form preincubated with plasminogen.

These features may be reflected <u>in vivo.</u>

There have been clinical trials for the dosage of SK complexed with plasminogen; one objective is to avoid the inhibition of SK at high concentrations; another objective is to mask the antigen site of SK by bonding with plasminogen in order to decrease antigenicity. Therefore, a reduction in the antigenicity of SAK is also expected in the case of the SAK-plasminogen complex.

SK is rapidly degraded once it is incubated with plasminogen (the molar ratio is about 1:1) and the plasminogen (or plasmin) gradually autodegrades, and therefore, it is known that SK cannot be administered in the form described above. Some experiments have been performed to acylate the active centre of the SK-plasminogen complex with the objectives of increasing its fibrin specificity by deacylating the active centre at the site of thrombus and of preventing the autodegradation of the complex. On the other hand, the activity of the SAK-plasminogen (or plasmin) complex in accordance with the present invention does not decrease at all even after incubation at 37 ° C for 24 hours, and some data have been obtained indicating that plasminogen bound to SAK is also resistant to autodegradation; the complex that SAK forms with plasminogen is sufficiently stable even if it is not acylated at its active centre.

Thus, the SAK-plasminogen (or plasmin) complex not only covers the defects of SAK but also has the advantage of stability compared with the SK-plasminogen complex.

Further, thrombolysis has been observed in dogs to which 0.002 mg of SAK was administered and, in the case of human therapy, 0.1-0.2 mg may be a sufficient dose if the above dose in dogs is approximately converted. This dose is comparable to about 1 mg of plasminogen in a molar conversion.

In addition, SK has a smaller molecular weight than the conventional thrombolytic agents and, therefore, it has an advantage in its permeability into thrombi.

<u>Staphylococcus aureus</u> has been used in the production of SAK conventionally and the production efficiency has been poor, accompanied by contamination toxicity; at present, the cloning in <u>Escherichia coli</u> has been used to enable the production of SAK on an industrial scale (Sato, T<u>: Eur. J. Biochem</u>., 149; 557-63, (1985)). Thus, SAK has an economic advantage, compared to other thrombolytic agents.

## Claims

### Claims for the following Contracting States : AT, BE, CH, DE, FR, GB, IT, LI, LU, NL, SE

1. A complex of staphylokinase and either plasminogen or plasmin for use in medicine.

2. A complex as claimed in claim 1 for use as a thrombolytic agent.

3. A pharmaceutical composition for use in medicine comprising a complex of staphylokinase and either plasminogen or plasmin, together with a pharmaceutically or veterinarily acceptable carrier.

4. A pharmaceutical composition for use in medicine as claimed in claim 3, wherein the complex is preparable by mixing and incubating staphylokinase and plasminogen.

5. A composition for use in medicine as claimed in claim 4, wherein the staphylokinase and plasminogen are mixed in a molar ratio of 1:1.

6. A composition for use in medicine as claimed in claim 4 or claim 5, wherein the incubation is carried out for one hour at room temperature.

7. The use of a complex of staphylokinase and either plasminogen or plasmin in the preparation of a thrombolytic agent.

### Claims for the following Contracting State : ES

1. A process for the preparation of a pharmaceutical composition comprising admixing a complex of staphylokinase and either plasminogen or plasmin with a pharmaceutically or veterinarily acceptable carrier.

2. A process as claimed in claim 1, wherein the complex is preparable by mixing and incubating staphylokinase and plasminogen.

3. a process as claimed in claim 2, wherein the staphylokinase and plasminogen are mixed in a molar ratio of 1:1.

4. A process as claimed in claim 2 or claim 3, wherein the incubation is carried out for one hour at room temperature.

5. The use of a complex of staphylokinase and either plasminogen or plasmin in the preparation of a thrombolytic agent.

**Patentansprüche**

**Patentansprüche für folgende Vertragsstaaten : AT, BE, CH, DE, FR, GB, IT, LI, LU, NL, SE**

1. Komplex aus Staphylokinase und entweder Plasminogen oder Plasmin zur Verwendung in der Medizin.

2. Komplex wie in Anspruch 1 beansprucht zur Verwendung als thrombolytisches Mittel.

3. Pharmazeutische Zusammensetzung zur Verwendung in der Medizin, umfassend einen Komplex aus Staphylokinase und entweder Plasminogen oder Plasmin zusammen mit einem pharmazeutisch oder veterinärmedizinisch annehmbaren Träger.

4. Pharmazeutische Zusammensetzung zur Verwendung in der Medizin wie in Anspruch 3 beansprucht, in welcher der Komplex durch Mischen und Inkubieren von Staphylokinase und Plasminogen herstellbar ist.

5. Zusammensetzung zur Verwendung in der Medizin wie in Anspruch 4 beansprucht, in welcher Staphylokinase und Plasminogen im Molverhältnis 1 : 1 gemischt sind.

6. Zusammensetzung zur Verwendung in der Medizin wie in Anspruch 4 oder 5 beansprucht, in welcher die Inkubation eine Stunde bei Raumtemperatur durchgeführt wird.

7. Verwendung eines Komplexes aus Staphylokinase und entweder Plasminogen oder Plasmin bei der Herstellung eines trhomobolytischen Mittels.

**Patentansprüche für folgenden Vertragsstaat : ES**

1. Verfahren zur Herstellung einer pharmazeutischen Zusammensetzung, umfassend das Vermischen eines Komplexes aus Staphylokinase und entweder Plasminogen oder Plasmin mit einem pharmazeutisch oder veterinärmedizinisch annehmbaren Träger.

2. Verfahren wie in Anspruch 1 beansprucht, bei welchem der Komplex durch Mischen und Inkubieren von Staphylokinase und Plasminogen herstellbar ist.

3. Verfahren wie in Anspruch 2 beansprucht, bei welchem Staphylokinase und Plasminogen im Molverhältnis 1 : 1 gemischt werden.

4. Verfahren wie in Anspruch 2 oder Anspruch 3 beansprucht, bei welchem die Inkubation eine Stunde bei Raumtemperatur durchgeführt wird.

5. Verwendung eines Komplexes aus Staphylokinase und entweder Plasminogen oder Plasmin bei der Herstellung eines thrombolytischen Mittels.

**Revendications**

**Revendications pour les Etats contractants suivants : AT, BE, CH, DE, FR, GB, IT, LI, LU, NL, SE :**

1. Complexe de staphylokinase et d'un plasminogène ou de plasmine pour usage en médecine.

2. Complexe selon la revendication 1, devant servir d'agent thrombolytique.

3. Composition pharmaceutique pour usage en médecine comprenant un complexe de staphylokinase et de plasminogène ou de plasmine ensemble avec un véhicule acceptable sur le plan pharmaceutique ou vétérinaire.

**4.** Composition pharmaceutique pour usage en médecine selon la revendication 3, dans laquelle on peut préparer le complexe en mélangeant et en incubant la staphylokinase et le plasminogène.

**5.** Composition pour usage en médecine selon la revendication 4, dans laquelle la staphylokinase et le plasminogène sont mélangés en un rapport molaire de 1:1.

**6.** Composition pour usage en médecine selon la revendication 4 ou 5, dans laquelle on effectue l'incubation pendant une heure à température ambiante.

**7.** Utilisation d'un complexe de staphylokinase et de plasminogène ou de plasmine pour la préparation d'un agent thrombolytique.

**Revendications pour l'Etat contract suivant : ES**

**1.** Procédé de préparation d'une composition pharmaceutique, qui consiste à mélanger un complexe de staphylokinase et de plasminogène ou de plasmine avec un véhicule acceptable sur le plan pharmaceutique ou vétérinaire.

**2.** Procédé selon la revendication 1, dans lequel on prépare le complexe en mélangeant et incubant la staphylokinase et le plasminogène.

**3.** Procédé selon la revendication 2, dans lequel on mélange la staphylokinase et le plasminogène en un rapport molaire de 1:1.

**4.** Procédé selon la revendication 2 ou 3, dans lequel on effectue l'incubation pendant une heure à température ambiante.

**5.** Utilisation d'un complexe de staphylokinase et de plasminogène ou de plasmine pour la préparation d'un agent thrombolytique.